(19) Europäisches Patentamt

European Patent Office

Office européen des brevets

(11) **EP 2 163 606 A1**

(12) **EUROPEAN PATENT APPLICATION**

(43) Date of publication:
**17.03.2010 Bulletin 2010/11**

(51) Int Cl.:
***C11D 3/386*** *(2006.01)*

(21) Application number: **09154901.4**

(22) Date of filing: **11.03.2009**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK TR**<br>Designated Extension States:<br>**AL BA RS** | (72) Inventor: **Lant, Neil Joseph**<br>**Newcastle upon Tyne, NE3 5RG (GB)** |
| | (74) Representative: **Howard, Phillip Jan**<br>**Procter & Gamble**<br>**Technical Centres Limited**<br>**Whitley Road**<br>**Longbenton**<br>**Newcastle upon Tyne NE12 9TS (GB)** |
| (30) Priority: **27.08.2008 US 190305 P** | |
| (71) Applicant: **The Procter and Gamble Company**<br>**Cincinnati, Ohio 45202 (US)** | |

(54) **A detergent composition comprising gluco-oligosaccharide oxidase**

(57) The present invention relates to a detergent composition comprising gluco-oligosaccharide oxidase.

SEQUENCE LISTING

<110>  THE PROCTER & GAMBLE COMPANY

<120>  A DETERGENT COMPOSITION COMPRISING GLUCO-OLIGOSACCHARIDE OXIDASE

<130>  CM3373F

<160>  3

<170>  PatentIn version 3.3

<210>  1
<211>  499
<212>  PRT
<213>  Acremonium strictum

<400>  1

Met Val Arg Ile Gln Glu Leu Thr Ala Ala Leu Ser Leu Ala Ser Val
1               5                   10                  15


Val Gln Ala Ser Trp Ile Gln Lys Arg Asn Ser Ile Asn Ala Cys Leu
                20                  25                  30


Ala Ala Ala Asp Val Glu Phe His Glu Glu Asp Ser Glu Gly Trp Asp
            35                  40                  45


Met Asp Gly Thr Ala Phe Asn Leu Arg Val Asp Tyr Asp Pro Ala Ala
        50                  55                  60


Ile Ala Ile Pro Arg Ser Thr Glu Asp Ile Ala Ala Ala Val Gln Cys
65                  70                  75                  80


Gly Leu Asp Ala Gly Val Gln Ile Ser Ala Lys Gly Gly Gly His Ser
                85                  90                  95


Tyr Gly Ser Tyr Gly Phe Gly Gly Glu Asp Gly His Leu Met Leu Glu
                100                 105                 110


Leu Asp Arg Met Tyr Arg Val Ser Val Asp Asp Asn Asn Val Ala Thr
            115                 120                 125


Ile Gln Gly Gly Ala Arg Leu Gly Tyr Thr Ala Leu Glu Leu Leu Asp
        130                 135                 140


Gln Gly Asn Arg Ala Leu Ser His Gly Thr Cys Pro Ala Val Gly Val
145                 150                 155                 160

Gly Gly His Val Leu Gly Gly Gly Tyr Gly Phe Ala Thr His Thr His
                    165                 170                 175

Gly Leu Thr Leu Asp Trp Leu Ile Gly Ala Thr Val Val Leu Ala Asp
                    180                 185                 190

Ala Ser Ile Val His Val Ser Glu Thr Glu Asn Ala Asp Leu Phe Trp
                    195                 200                 205

Ala Leu Arg Gly Gly Gly Gly Gly Phe Ala Ile Val Ser Glu Phe Glu
                    210                 215                 220

Phe Asn Thr Phe Glu Ala Pro Glu Ile Ile Thr Thr Tyr Gln Val Thr
225                 230                 235                 240

Thr Thr Trp Asn Arg Lys Gln His Val Ala Gly Leu Lys Ala Leu Gln
                    245                 250                 255

Asp Trp Ala Gln Asn Thr Met Pro Arg Glu Leu Ser Met Arg Leu Glu
                    260                 265                 270

Ile Asn Ala Asn Ala Leu Asn Trp Glu Gly Asn Phe Phe Gly Asn Ala
                    275                 280                 285

Lys Asp Leu Lys Lys Ile Leu Gln Pro Ile Met Lys Lys Ala Gly Gly
                    290                 295                 300

Lys Ser Thr Ile Ser Lys Leu Val Glu Thr Asp Trp Tyr Gly Gln Ile
305                 310                 315                 320

Asn Thr Tyr Leu Tyr Gly Ala Asp Leu Asn Ile Thr Tyr Asn Tyr Asp
                    325                 330                 335

Val His Glu Tyr Phe Tyr Ala Asn Ser Leu Thr Ala Pro Arg Leu Ser
                    340                 345                 350

Asp Glu Ala Ile Gln Ala Phe Val Asp Tyr Lys Phe Asp Asn Ser Ser
                    355                 360                 365

Val Arg Pro Gly Arg Gly Trp Trp Ile Gln Trp Asp Phe His Gly Gly
                    370                 375                 380

```
Lys Asn Ser Ala Leu Ala Ala Val Ser Asn Asp Glu Thr Ala Tyr Ala
385                 390                 395                 400


His Arg Asp Gln Leu Trp Leu Trp Gln Phe Tyr Asp Ser Ile Tyr Asp
                405                 410                 415


Tyr Glu Asn Asn Thr Ser Pro Tyr Pro Glu Ser Gly Phe Glu Phe Met
                420                 425                 430


Gln Gly Phe Val Ala Thr Ile Glu Asp Thr Leu Pro Glu Asp Arg Lys
            435                 440                 445


Gly Lys Tyr Phe Asn Tyr Ala Asp Thr Thr Leu Thr Lys Glu Glu Ala
        450                 455                 460


Gln Lys Leu Tyr Trp Arg Gly Asn Leu Glu Lys Leu Gln Ala Ile Lys
465                 470                 475                 480


Ala Lys Tyr Asp Pro Glu Asp Val Phe Gly Asn Val Val Ser Val Glu
                485                 490                 495


Pro Ile Ala
```

```
<210>  2
<211>  492
<212>  PRT
<213>  Pyrenophora tritici-repentis Pt-1C-BFP

<400>  2

Met Val Lys Ile Tyr Ser Glu Trp Leu Tyr Thr Ala Thr Ala Phe Phe
1               5                   10                  15


Ala Leu Ser Ser Ala Thr Leu Asp Arg Arg Ala Ala Val Asp Asp Cys
            20                  25                  30


Leu Lys Ser His Gln Val Pro Val Tyr Ala Ala Gly Thr Ala Asn Tyr
        35                  40                  45


Thr Gln Ala Ile Lys Pro Phe Asn Leu Arg Leu Ser Phe Thr Pro Ala
        50                  55                  60


Ser Tyr Ala Val Pro Gln Thr Ile Lys His Ile Gln Asp Ala Val Ala
```

|      |      | 65   |      |      |      | 70   |      |      |      | 75   |      |      |      | 80   |
|------|------|------|------|------|------|------|------|------|------|------|------|------|------|------|

Cys Gly Val Ala Asn Lys Ile Pro Val Thr Ala Lys Cys Gly Gly His
                85                  90                  95

Ser Tyr Ala Ala His Gly Leu Gly Gly Glu Asn Ala His Leu Ile Val
                100                 105                 110

Asp Met Gln Arg Phe Asn Ser Val Thr Val Asp Gln Gln Ala Gln Arg
                115                 120                 125

Ala Val Val Gln Ala Gly Gly Arg Leu Gly Asn Ile Ala Leu Ala Leu
        130                 135                 140

Tyr Asp Gln Gly Lys Gln Ala Ile Ser His Gly Thr Cys Pro Gly Val
145                 150                 155                 160

Gly Val Ser Gly Leu Thr Leu His Gly Gly Tyr Gly Leu Ser Ser Arg
                165                 170                 175

Lys His Gly Leu Ala Leu Asp Asn Val Val Ser Ala Thr Val Val Leu
                180                 185                 190

Ala Asn Ser Thr Val Val Thr Ala Ser Ala Asp Ser Asn Gln Asp Leu
                195                 200                 205

Phe Trp Ala Leu Arg Gly Ala Gly Ala Ala Phe Gly Ile Val Val Asp
        210                 215                 220

Phe Thr Phe Lys Thr Phe Thr Pro Leu Glu Ser Asn Val Ile Phe Asp
225                 230                 235                 240

Tyr Ser Leu Ser Pro Lys Asn Thr Ser Gln Leu Ala Lys Tyr Val Thr
                245                 250                 255

Ala Leu Gln Asp Phe Ser Ile Asn Asp Gln Pro Ala Glu Leu Asp Met
                260                 265                 270

Arg Met Phe Leu Pro Lys Gln Leu Thr Gly Val Tyr His Gly Asn Arg
        275                 280                 285

Ser Glu Phe Asp Lys Val Met Ala Pro Leu Leu Ala Lys Leu Asp Ile
        290                 295                 300

```
Pro Ala Gly Ser Gly Lys Val Ser Val Lys Gly Trp Ile Asp Thr Leu
305             310             315             320


Thr His Phe Ala Phe Gly Pro Leu Lys Gln Ala Glu Val Tyr Asp Thr
                325             330             335


His Glu Asn Phe Tyr Ala Lys Ser Leu Met Pro Glu Ala Leu Ser Pro
                340             345             350


Ala Ala Ile Lys Ala Met Ser Asp Tyr Tyr Phe Thr Thr Ala Ser Lys
        355             360             365


Ile Thr Arg Gly Trp Tyr Leu Leu Ile Asp Leu His Gly Gly Lys Ser
    370             375             380


Ala Ser Ile Ser Gln Val Gly Pro Asp Glu Thr Ser Tyr Ser His Arg
385             390             395             400


Lys Ser Ile Phe Lys Met Gln Phe Tyr Asp Arg Ile Phe Pro Asp Asn
                405             410             415


Ala Thr Tyr Lys Pro Glu Tyr Met Ser Phe Leu Asn Gly Trp Val Asn
                420             425             430


Ala Ile Glu Asp Ala Ser Asn Gly Ser Lys Tyr Gly Met Tyr Val Asn
        435             440             445


Tyr Ala Asp Thr Gly Leu Ser Arg Thr Glu Ala His Ser Arg Tyr Trp
    450             455             460


Gly Glu Asn Tyr Asp Arg Leu Val Lys Ile Lys Lys Ser Leu Asp Pro
465             470             475             480


Asn Asn Val Phe Glu Gly Pro Gln Leu Val Gly Ser
                485             490


<210>  3
<211>  497
<212>  PRT
<213>  Laccaria bicolor S238N-H82

<400>  3
```

```
Met Ala His Arg Leu Leu Phe Phe Ala Phe Leu Thr Leu Trp Ser Ser
1               5                   10                  15

Leu Ala Thr Ala Ser Thr Ala Asp Leu Val Ser Ser Leu Lys Ala Leu
            20                  25                  30

Asn Phe Thr Ile Val Thr Pro Gly Asp Pro Asn Tyr Pro Thr Ala Ser
            35                  40                  45

Thr Ala Phe Asn Leu Arg Tyr Thr Phe Glu Pro Ala Ala Val Ala Tyr
    50                  55                  60

Pro Thr Asn Ala Gln Gln Ile Ser Ala Leu Leu Lys Val Ser Arg Gln
65                  70                  75                  80

Phe Asn His Gln Val Val Ala Arg Ser Gly Gly His Ser Tyr Ile Ala
            85                  90                  95

Asn Gly Leu Gly Gly Lys Asp Gly Val Val Val Asp Met Thr Asn
            100                 105                 110

Phe Met Thr Val Ala Val Asp Ser Ser Thr Gly Thr Ala Ile Ile Gly
        115                 120                 125

Pro Gly Asn Arg Leu Gly Asn Val Ala Leu Ala Leu Asn Asp Lys Gly
    130                 135                 140

Arg Ala Leu Pro His Gly Thr Cys Pro Tyr Val Gly Val Gly Gly His
145                 150                 155                 160

Ser Gly Phe Gly Gly Tyr Gly Phe Thr Ser Arg Lys Trp Gly Leu Leu
            165                 170                 175

Leu Asp Thr Ile Leu Ser Ile Asn Val Val Leu Ala Asp Gly Thr Ile
            180                 185                 190

Ala Asn Val Ser Gln Thr Thr His Pro Asn Leu Phe Trp Ala Leu Arg
        195                 200                 205

Gly Ser Ser Ala Ser Phe Gly Ile Val Thr Ala Ile Gln Val Met Thr
    210                 215                 220

Phe Pro Thr Pro Ser Ser Ala Thr Val Phe Glu Tyr Asn Trp Asn Leu
```

|     | 225 |     |     |     | 230 |     |     |     | 235 |     |     |     | 240 |
|-----|-----|-----|-----|-----|-----|-----|-----|-----|-----|-----|-----|-----|-----|

Asn Ala Ala Asp Ala Ala Lys Ala Val Gly Ala Phe Gln His Phe Val
        245                     250                 255

Gln Thr Asn Ile Pro Gln Glu Phe Ala Ala Glu Ile Val Ile Gly Lys
        260                     265                 270

Gly Ser Val Gln Gly Asn Leu Ser Trp Gly Leu Thr Gly Gly Trp Tyr
        275                     280                 285

Gly Pro Ala Asp Gln Phe Gln Ser Val Ile Ala Pro Phe Leu Ala Lys
        290                     295                 300

Val Pro Lys Pro Ala Ser Thr Ser Leu Thr Pro Gly Thr Tyr Ile Asn
305                     310                 315                 320

Ser Val Gln Phe Leu Gly Gly Leu Gly Arg Leu Asn Thr Ser Ser Ser
                325                     330                 335

Pro Asp Gly His Asp Thr Phe Tyr Ala Lys Ser Leu Met Thr Pro Glu
                340                     345                 350

Ala Ser Pro Met Ser Asn Ala Ala Arg Thr Ala Phe Met Ser Tyr Leu
                355                     360                 365

Ala Asn Asp Gly Phe Asn Ala Thr Thr Asp Trp Phe Val Glu Ile Glu
        370                     375                 380

Leu Tyr Gly Gly Thr Asn Ser Ala Ile Asn Asn Val Pro Ile Asp Ala
385                     390                 395                 400

Thr Ala Phe Ala His Arg Ser Ser Met Phe Thr Ile Gln Phe Tyr Thr
                405                     410                 415

Ser Ala Pro Gly Asn Val Pro Pro Phe Pro Pro Ala Gly Phe Ser Phe
                420                     425                 430

Leu Asp Glu Met Val Asn Ser Ile Val Asn Asn Ser Pro Lys Gly Trp
        435                     440                 445

Asp Tyr Gly Ala Tyr Ala Asn Tyr Ile Asp Asp Arg Leu Gln Asn Trp
        450                     455                 460

11

```
Gln Gln Leu Tyr Tyr Gly Pro His Tyr Pro Arg Leu Lys Ala Leu Lys
465                 470                 475                     480


Asp Gln Tyr Asp Pro Met Asn Thr Phe Ser Phe Pro Thr Ser Ile Glu
                485                 490                 495


Glu
```

**Description**

FIELD OF THE INVENTION

The present invention relates to detergent compositions comprising gluco-oligosaccharide oxidase. The compositions of the present invention exhibit good bleaching performance. Preferably, the compositions are laundry detergent compositions, most preferably liquid laundry detergent compositions.

BACKGROUND OF THE INVENTION

The incorporation of bleach into a detergent composition requires a carefully balance of having good bleaching performance and good bleach stability, especially good product storage stability. Whilst many different types of bleach have been considered for incorporation into detergent compositions, such as pre-formed peracids, transition metal bleach catalysts, nitrile quaternary amine bleach activators, cationic imine bleach boosting compounds and the like, there remains very few bleach technologies that exhibit sufficient bleach stability, especially good product storage stability, whilst at the same time providing good bleaching performance and acceptable fabric integrity profile. This is especially true for liquid laundry detergent compositions. Even after decades of development, the commercial yardstick for bleach profile in a solid laundry detergent composition remains, with very few exceptions, percarbonate and/or perborate sources of hydrogen peroxide in combination with bleach activators such as tetraacetylethylenediamine (TAED) and/or alkyloxybenezenesulphonate (AOBS).

With the recent trend in detergent formulation towards increased reliance on improved weight-efficient and cost-effective technologies, developments of bleach catalysis has resurfaced and bleach catalyst technologies such as transition metal catalysts and cationic imine bleach boosting compounds have rekindled. However, these bleach catalyst technologies are used in relatively very small amounts, due to their high weight efficiency.

Using bleach catalysts at these very low levels means that their activity is easily depleted by other components of the detergent composition, such as polyamines. Further instability can arise if the catalysts prematurely activate during storage. This is especially true when the detergent composition is a laundry detergent composition due to the complexity and numerous other chemical components present therein. Also, the bleach stability of the bleach catalyst, and the need to avoid any bleach depletion, is even more critical when the detergent composition is in liquid form, such as a liquid laundry detergent composition.

In addition, using bleach catalysts at very low levels means that it is important that the catalysts are active in the wash when and where bleaching performance is needed. This typically means at the surface to be cleaned. If the bleach catalyst does not reach the surface to be cleaned in any appreciable amount but instead remains in the wash liquor and catalyses bleaching performance in the wash solution, this solution bleaching performance is usually not very efficient as the majority of the available oxygen will be used to bleach soils that are already suspended in the wash liquor and only a minority of the available oxygen will be used to bleach soils that are present at the surface and contribute to the cleaning performance of the detergent composition. This is especially true when the composition is a laundry detergent composition and the surface to be cleaned is a fabric surface.

The Inventor has overcome the above problems and has found a bleach catalyst enzyme that has good stability profile, provides excellent bleaching performance, and exhibits very good bleach efficiency. The inventor has found that a specific gluco-oligosaccharide oxidase generates available oxygen at the fabric surface during a laundering process: the substrate for this specific bleaching enzyme are oligosaccharides arising from cotton as well as other reducing sugars, such as glucose and lactose, present in the fabric stains.

The Inventor has also found that this enzyme provides excellent whiteness benefits due to its effect on the cotton fabric surface which makes the cotton more resistant to soil re-deposition. Without wishing to be bound by theory, the Inventor believes the enzymes acts so as to make the cotton surface more negatively charged (e.g. the enzyme reacts with the terminal glucose moieties of amorphous cellulose and other oligosaccharides of cotton) by oxidizing the terminal reducing ends to a gluconic acid form. This increases the negative charge of the cotton, leading to improved soil repulsion and improved soil anti-redeposition properties of the modified cotton.

SUMMARY OF THE INVENTION

The present invention provides a composition according to the claims.

DETAILED DESCRIPTION OF THE INVENTION

Detergent composition

The detergent composition can be any detergent composition, such as a laundry detergent composition, dishwashing detergent composition, hard-surface cleaning detergent composition, and the like. Preferably, the composition is a laundry detergent composition.

The composition can be in any form, such as a solid or a liquid. Solid forms typically include granular, flake, noodle, needle and the like. Alternatively the composition can in be the form of a gel, paste, suspension or the like. Preferably, the composition is in the form of a liquid. Most preferably, the composition is a liquid laundry detergent composition.

The composition typically comprises detergent adjunct ingredients.

Gluco-oligosaccharide oxidase

The composition comprises gluco-oligosaccharide oxidase. Preferably, the composition comprises from 0.00015wt% to 0.50wt% gluco-oligosaccharide oxidase.

Preferably, the gluco-oligosaccharide oxidase is a parent or variant of the gluco-oligosaccharide oxidase derived from organisms selected from: *Acremonium strictum*, *Pyrenophora tritici-repentis* Pt-1C-BFP and *Laccaria bicolor* S238N-H82.

A suitable gluco-oligosaccharide oxidase is derived from Acremonium strictum described in more detail in M.-H.Lee et al, Appl. Environ. Microbiol., 71 (12), 8881-8887 (2005). The amino acid sequence of this gluco-oligosaccharide oxidase is given in sequence I.D. No. 1.

The gluco-oligosaccharide oxidase preferably has an amino acid sequence that is at least 45wt%, or at least 50wt%, or at least 55wt%, or at least 60wt%, or at least 65wt%, or at least 70wt%, or at least 75wt%, or at least 80wt%, or at least 85wt%, or at least 90wt%, or at least 95wt% identity to sequence I.D. 1. Preferably the gluco-oligosaccharide oxidase has an amino acid sequence of sequence I.D. 1.

Another suitable gluco-oligosaccharide oxidase is derived from *Pyrenophora tritici-repentis* Pt-1C-BFP according to the NCBI Reference Sequence: XP_001938664.1

The amino acid sequence of this gluco-oligosaccharide oxidase is given in sequence I.D. No. 2.

The gluco-oligosaccharide oxidase preferably has an amino acid sequence that is at least 40wt%, or at least 45wt%, or at least 50wt%, or at least 55wt%, or at least 60wt%, or at least 65wt%, or at least 70wt%, or at least 75wt%, or at least 80wt%, or at least 85wt%, or at least 90wt%, or at least 95wt% identity to sequence I.D. 2. Preferably the gluco-oligosaccharide oxidase has an amino acid sequence of sequence I.D. 2.

Another suitable gluco-oligosaccharide oxidase is derived from *Laccaria bicolor S238N-H82* according to the NCBI Reference Sequence: XP_001889181.1. The genome of which is described in more detail in F. Martin et al, Nature, 452 (7183), 88-92 (2008).

The amino acid sequence of this gluco-oligosaccharide oxidase is given in sequence I.D. No. 3.

The gluco-oligosaccharide oxidase preferably has an amino acid sequence that is at least 35wt%, or at least 40wt%, or at least 45wt%, or at least 50wt%, or at least 55wt%, or at least 60wt%, or at least 65wt%, or at least 70wt%, or at least 75wt%, or at least 80wt%, or at least 85wt%, or at least 90wt%, or at least 95wt% identity to sequence I.D. 3. Preferably the gluco-oligosaccharide oxidase has an amino acid sequence of sequence I.D. 3.

The amino acid sequence identity is the relatedness between two amino acid sequences described by the parameter "identity". For purposes of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277; http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(Identical\ Residues \times 100)/(Length\ of\ Alignment - Total\ Number\ of\ Gaps\ in\ Alignment)$$

EXAMPLES

Example 1

A liquid laundry detergent composition comprises: 0.05wt% gluco-oligosaccharide oxidase[*], 15wt% detersive surfactant, 3wt% fatty acid, 0.3wt% chelant, 0.2wt% brightener, 5wt% solvent, 0.5wt% perfume, buffer to pH 8.2, water and miscellaneous to 100wt%.

Example 2

A solid laundry detergent composition comprises: 0.05wt% gluco-oligosaccharide oxidase[*], 10wt% detersive surfactant, 10wt% sodium carbonate, 0.3wt% chelant, 0.2wt% brightener, 0.5wt% perfume, moisture, sodium sulphate and miscellaneous to 100wt%.

The gluco-oligosaccharide oxidase is the wildtype from Acremonium strictum prepared according to the method described in M.-H.Lee et al, Appl. Environ. Microbiol., 71 (12), 8881-8887 (2005).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. A detergent composition comprising gluco-oligosaccharide oxidase.

2. A detergent composition according to claim 1, wherein the gluco-oligosaccharide oxidase is a parent or variant of the gluco-oligosaccharide oxidase derived from *Acremonium strictum*, *Pyrenophora tritici-repentis* Pt-1C-BFP or *Laccaria bicolor* S238N-H82.

3. A detergent composition according to any preceding claim, wherein the gluco-oligosaccharide oxidase has an amino acid sequence that is at least 50% identity to any of sequence I.D. 1,2 or 3.

4. A composition according to any preceding claim, wherein the gluco-oligosaccharide oxidase has an amino acid sequence that is at least 70% identity to any of the sequence I.D. 1, 2 or 3.

5. A composition according to any preceding claim, wherein the gluco-oligosaccharide oxidase has an amino acid sequence of any of the sequence I.D. 1, 2 or 3.

6. A composition according to any preceding claim, wherein the composition is a laundry detergent composition.

7. A composition according to any preceding claim, wherein the composition is in liquid form.

8. A composition according to any preceding claim, wherein the composition is in solid form.

9. A composition according to any preceding claim, wherein the composition comprises detersive surfactant.

Europäisches
Patentamt

European
Patent Office

Office européen
des brevets

## EUROPEAN SEARCH REPORT

Application Number

EP 09 15 4901

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/131503 A (NOVOZYMES AS [DK]; LAURIDSEN CARSTEN [DK]; NIELSEN JESPER DUUS [DK]) 14 December 2006 (2006-12-14) | 1-9 | INV. C11D3/386 |
| Y | * paragraphs [0001], [0005], [0013], [0014]; claims 1-4 * | 1-9 | |
| X | EP 1 041 890 B1 (NOVONORDISK AS [DK] NOVOZYMES AS [DK]) 20 April 2005 (2005-04-20) * paragraph [0120] * | 1-9 | |
| D,Y | LEE M-H ET AL: "Structural characterization of glucooligosaccharide oxidase from Acremonium strictum" APPLIED AND ENVIRONMENTAL MICROBIOLOGY DECEMBER 2005 AMERICAN SOCIETY FOR MICROBIOLOGY US, vol. 71, no. 12, December 2005 (2005-12), pages 8881-8887, XP002529148 * the whole document * | 1-9 | |

TECHNICAL FIELDS
SEARCHED       (IPC)

C11D

The present search report has been drawn up for all claims

| Place of search | Date of completion of the search | Examiner |
|---|---|---|
| Munich | 6 August 2009 | Klier, Erich |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 15 4901

This annex lists the patent family members relating to the patent documents cited in the above-mentioned European search report.
The members are as contained in the European Patent Office EDP file on
The European Patent Office is in no way liable for these particulars which are merely given for the purpose of information.

06-08-2009

| Patent document cited in search report | | Publication date | Patent family member(s) | | Publication date |
|---|---|---|---|---|---|
| WO 2006131503 | A | 14-12-2006 | NONE | | |
| EP 1041890 | B1 | 20-04-2005 | AT | 293359 T | 15-05-2005 |
| | | | AU | 753578 B2 | 24-10-2002 |
| | | | AU | 1751899 A | 12-07-1999 |
| | | | CA | 2314996 A1 | 01-07-1999 |
| | | | CN | 1379989 A | 20-11-2002 |
| | | | CN | 1283082 A | 07-02-2001 |
| | | | DE | 69829878 D1 | 25-05-2005 |
| | | | DE | 69829878 T2 | 02-03-2006 |
| | | | WO | 9931990 A1 | 01-07-1999 |
| | | | EP | 1041890 A1 | 11-10-2000 |
| | | | ES | 2241189 T3 | 16-10-2005 |
| | | | JP | 2001526058 T | 18-12-2001 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M.-H.Lee et al.** *Appl. Environ. Microbiol.,* 2005, vol. 71 (12), 8881-8887
- **F. Martin et al.** *Nature,* 2008, vol. 452 (7183), 88-92
- **Needleman ; Wunsch.** *J. Mol. Biol.,* 1970, vol. 48, 443-453
- **Rice et al.** *Trends in Genetics,* 2000, vol. 16, 276-277, http://emboss.org